# EUROPÄISCHE PATENTSCHRIFT

(11) **EP 0 583 702 B1**
(45) Veröffentlichungstag und Bekanntmachung des Hinweises auf die Patenterteilung: **25.03.1998**
(21) Anmeldenummer: 93112621.3
(22) Anmeldetag: 06.08.1993
(51) Int. Cl.: C11D 1/90, A61K 7/08, A61K 7/075, A61K 7/50, D06M 13/342

(54) **Wässrige Zubereitungen, die Betaine auf Basis polymerer Fettsäuren enthalten**
Aqueous preparations containing polymeric fatty acid based betaines
Préparations aqueuses contenant des bétaines à base d'acides gras polymères

(30) Priorität: 19.08.1992 DE 4227391
(43) Veröffentlichungstag der Anmeldung: 23.02.1994
(73) Patentinhaber: Th. Goldschmidt AG, 45127 Essen (DE)
(72) Erfinder: Grüning, Burghard, Dr., D-45134 Essen (DE)

(56) Entgegenhaltungen:
- EP-A- 0 077 674
- DE-A- 1 617 497
- DE-B- 1 062 392
- CHEMICAL ABSTRACTS, vol. 112, no. 4, 22. Januar 1990, Columbus, Ohio, US; abstract no. 22799a, KROB VACLAV ET AL. Seite 113 ; & CS-A-260 933 14. April 1989

## Beschreibung

Betaine der allgemeinen Formel 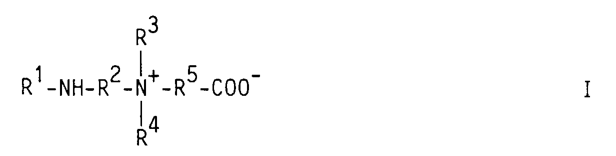 wobei
- R¹: der Acylrest einer Fettsäure mit 8 bis 18 Kohlenstoffatomen und
- R²: ein zweiwertiger aliphatischer Kohlenwasserstoffrest mit 2 bis 5 Kohlenstoffatomen ist,
- R³, R⁴: unabhängig voneinander aliphatische Kohlenwasserstoffreste mit 1 bis 4 Kohlenstoffatomen sind und
- R⁵: der Rest -CH₂- oder -(CH₂)₂- ist,
sind seit langem bekannt. Sie sind in der Patentliteratur beschrieben, von der als Beispiel die DE-AS 10 62 393 angeführt wird.

Die Betaine werden als amphotere Tenside insbesondere für Haar- und Hautreinigungspräparate, wie Shampoos, hautschonende Schaum- und Duschgele, Intim- und Körperpflegemittel eingesetzt. Sie verbessern die dermatologischen Eigenschaften anionischer Tenside und bewirken ein angenehmes Hautgefühl. Darüber hinaus können die Betaine als Tenside für technische Zwecke, z.B. in der Textilindustrie oder als Korrosionsschutzmittel in Metallbehandlungsflüssigkeiten eingesetzt werden.

Die Betaine des Standes der Technik zeichnen sich durch eine hohe Schaumbildung aus, wobei der Schaum sich auch gegenüber Härtebildnern des Wassers stabil erweist. So erwünscht diese Schaumbildungstendenz für manche Anwendungszwecke sein kann, stört sie doch bei manchen Verwendungen, so daß insbesondere beim Einsatz der Betaine für technische Zwecke die Bereitstellung von schaumarmen Betainen erwünscht ist.

Aus der CS-PS 260933 sind Betaine bekannt, deren Fettsäurerest sich von dimeren und trimeren Fettsäuren ableitet. Derartige polymere Fettsäuren sind unter der Bezeichnung Dimer- und Trimersäuren im Handel erhältlich. Bei den Dimersäuren handelt es sich um ein Gemisch von Dicarbonsäuren mit insgesamt meist 36 Kohlenstoffatomen, die durch kovalente Verknüpfung ungesättigter Fettsäuren mit 18 Kohlenstoffatomen bei Temperaturen oberhalb 200°C, meist in Gegenwart von Tonmineralien, hergestellt werden. Als ungesättigte Fettsäuren werden im allgemeinen Ölsäure, Linolsäure und Linolensäure verwendet. Die auf diese Weise gewonnenen Dimersäuren können anschließend hydriert werden.

Zur Herstellung und Verwendung von Dimersäuren und deren physikalische und chemische Eigenschaften wird auf die Veröffentlichung "The Dimer Acids: The chemical and physical properties, reactions and applications", Ed. E.C. Leonard; Humko Sheffield Chemical, 1975, Memphis, Tenn., verwiesen.

Die Dimersäure kann folgender Formel entsprechen:

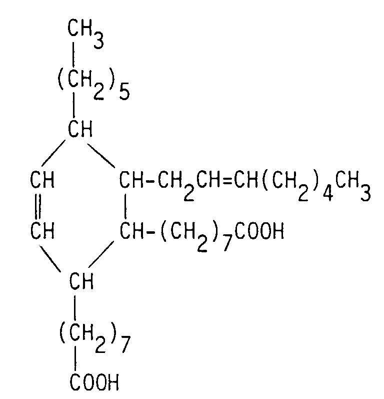

Die Trimersäuren werden in analoger Weise hergestellt und sind durch Trimerisation ungesättigter Fettsäure erhältliche Tricarbonsäuren mit meist 54 Kohlenstoffatomen.

Diese Polymersäuren sind in verschiedenen Reinheitsgraden im Handel erhältlich, denn das rohe Reaktionsprodukt, wie es sich aus der Dimerisierung ungesättigter C₁₈-Fettsäuren ergibt, enthält neben Dimersäure Trimersäure der monomeren Carbonsäuren und beträchtliche Mengen von Zwischenprodukten unbekannter Struktur im Molekulargewichtsbereich von C₁₈ bis C₃₆. Stellt man Betaine unter Einsatz der Polymersäuren her, stellt man fest, daß mit steigendem Reinheitsgrad der Polymersäuren die Viskosität ihrer wäßrigen Lösungen bei vergleichbarer Konzentration stark zunimmt, bis sie schließlich gelartig und nicht mehr fließfähig werden. So ist es möglich, bei Verwendung von rohen Dimer- und Trimersäure-Gemischen wäßrige Betainlösungen mit einem Festkörpergehalt von etwa 15 bis 25 Gew.-% herzustellen. Verwendet man jedoch gereinigte, insbesondere destillierte Dimersäuren, werden die wäßrigen Lösungen bereits bei Festkörpergehalten oberhalb von etwa 4 bis 5 Gew.-% fest. Dies schränkt die Verwendbarkeit solcher Betaine auf Dimersäurebasis ein. Insbesondere in kosmetischen Anwendungen, aber auch in zahlreichen technischen Anwendungen, wie z.B. der Behandlung von Textilien, ist die Verwendung möglichst sauberer, wohl definierter Substanzen anzustreben, um toxikologische Risiken möglichst gering zu halten, so daß gerade Betainen auf Basis gereinigter polymerer Fettsäuren besondere Bedeutung zukommt.

Man hat bereits Betaine, die unter Verwendung verschiedener Fettsäuren getrennt hergestellt worden waren, nach ihrer Herstellung vermischt, um bestimmte anwendungstechnische Eigenschaften zu erhalten. So ist in der EP-A2 0 121 791 eine Zubereitung für kosmetische Formulierungen beschrieben, welche eine Mischung von Cocoamidopropylbetainen und Oleamidopropylbetainen im Verhältnis 1 : 4 bis 3 : 2 enthält. Dieses Betain-Gemisch hat in wäßrigen kosmetischen Zubereitungen verdickende und schaumverstärkende Eigenschaften und zeigt verbesserte Emulgierfähigkeit z.B. für Jojoba-Öl.

Die vorliegende Erfindung befaßt sich mit dem technischen Problem, wäßrige Lösungen von Betainen auf Basis von Dimersäuren herzustellen, die auch bei höheren Konzentrationen noch flüssig sind. Die wäßrigen Zubereitungen sollen möglichst schaumarm sein, damit diese Polymersäurebetaine insbesondere auch für technische Anwendungszwecke eingesetzt werden können.

Überraschenderweise wurde nun gefunden, daß diese Eigenschaften bei einer erfindungsgemäßen Zubereitung zu finden sind, welche gekennzeichnet ist durch einen Gehalt von
- 5 bis 30 Gew.-%: eines Gemisches von Betainen, welches aus 15 bis 60 Gew.-% Betainen der allgemeinen Formel 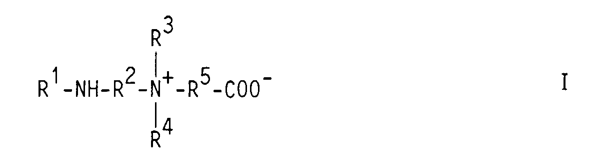 und
85 bis 40 Gew.-% Betainen der allgemeinen Formel 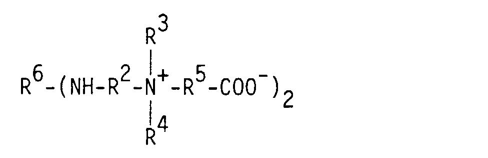 besteht und wobei die Gew.-% sich zu 100 Gew.-% ergänzen, wobei
R¹ der Acylrest einer Fettsäure mit 8 bis 18 Kohlenstoffatomen oder eines Gemisches dieser Fettsäuren ist,
R² ein zweiwertiger aliphatischer Kohlenwasserstoffrest mit 2 bis 5 Kohlenstoffatomen ist,
R³, R⁴ unabhängig voneinander aliphatische Kohlenwasserstoffreste mit 1 bis 4 Kohlenstoffatome sind,
R⁵ der Rest -CH₂- oder -(CH₂)₂- ist,
R⁶ ein von destillierter Dimersäure abgeleiteter Acylrest ist,
- 95 bis 70 Gew.-%: Wasser, Alkalisalze und gegebenenfalls übliche Zusatzstoffe wie Hilflösungsmittel, Konservierungsmittel oder dergl.

R¹ ist vorzugsweise der Acylrest eines Fettsäuregemisches mit im Durchschnitt etwa 12 bis 14 Kohlenstoffatomen, wie etwa das Gemisch der Cocosfettsäure.

R² ist vorzugsweise ein Alkylenrest der Formel -(CH₂)₂- oder -(CH₂)₃-.
R³ und R⁴ sind vorzugsweise Methylreste.
R⁵ ist vorzugsweise der Rest -CH₂-.
R⁶ ist der Acylrest destillierter Dimersäuren. Besonders bevorzugt sind destillierte Dimersäuren, wie sie z.B. unter der Bezeichnung PRIPOL 1012/1013 und in hydrierter Form unter der Bezeichnung PRIPOL 1008/1009 (von der Firma Unichema) im Handel erhältlich sind. Sie enthalten nur geringe Anteile (< 0,1 Gew.-%) an monobasischen und (< 5 Gew.-%) trimeren Säuren und weisen, insbesondere in hydrierter Qualität, eine helle Farbe auf. In diesem Falle hat der Index x einen Wert von 2. Man kann natürlich auch Dimersäuren verwenden, deren Gehalt an Trimersäure höher ist. Jedoch sinkt dann die Grenzkonzentration, von der ab die wäßrigen Lösungen der Betain-Gemische nicht mehr fließfähig sind.

Erfindungsgemäße Zubereitungen, bei denen R¹ der Acylrest eines Co-cosfettsäuregemisches und R⁶ der Acylrest einer destillierten Dimersäure ist, sind innerhalb der im Patentanspruch 1 angegebenen Grenzen bei einem Festkörpergehalt von bis zu 30 Gew.-% noch fließfähig. Die Zubereitungen sind schaumarm und deshalb für technische Zwecke besonders brauchbar.

Die erfindungsgemäßen Zubereitungen können für viele technische Zwecke eingesetzt werden. Eine bevorzugte Verwendung ist der Einsatz in der Textilindustrie. Die Betain-Gemische zeigen hohe Substantivität auf Fasern. Bei der Anwendung auf Wolle wird deren Haftlänge vergrößert. Unter Haftlänge versteht man dabei die Länge eines Wollfadens, ab der der Faden durch sein Eigengewicht reißt.

Die verminderte Schaumbildung macht den Einsatz der erfindungsgemäßen Betain-Gemische in Wasch- und Reinigungsmitteln, die zum Einsatz in Waschmaschinen bestimmt sind, möglich. Die erfindungsgemäßen Betain-Gemische verbessern insgesamt die Eigenschaften von Waschmitteln, indem sie insbesondere das Ausbluten von Farbstoffen vermindern und den Griff der gewaschenen Textilien verbessern.

Die erfindungsgemäßen Gemische eignen sich auch für andere technische Anwendungszwecke. Sie können als Dispergiermittel für Pigmente in Farben, Lacken und Anstrichmitteln eingesetzt werden und verhindern oder vermindern die Tendenz der Pigmente, sich in derartigen Produkten abzusetzen oder erleichtern das Wiederaufrühren der Pigmente. In Metallbehandlungsflüssigkeiten, wie Bohr- und Schneidöl-Emulsionen, verringern sie die Korrosivität der wäßrigen Zubereitungen. Sie können außerdem in vielfältiger Weise als Antistatika eingesetzt werden.

Andere Verwendungsmöglichkeiten sind in kosmetischen Zubereitungen gegeben. So bewirken die Betain-Gemische eine Konditionierung der Haare, wenn sie z.B. aus einem Shampoo oder einer Fönlotion angewendet werden. Das Haar erhält dadurch einen angenehmen Griff und eine verbesserte Festigkeit. Die Betain-Gemische sind Tensidzubereitungen mit einem sehr niedrigen Reizpotential. Sie eignen sich daher zur Bereitung besonders milder kosmetischer Formulierungen. Bei der Verwendung der erfindungsgemäßen Betain-Gemische in Haar- und Körperreinigungsmitteln zeigt sich, daß die Viskosität der wäßrigen Zubereitungen, bestehend aus anionaktiven Tensiden, wie Natriumlaurylethersulfat, und Elektrolyten, wie Alkalichloriden, stärker als mit herkömmlichen Betainen erhöht werden kann. Dies bedeutet aber auch, daß die gleiche Viskosität mit geringeren Zusätzen von anionaktiven Tensiden und/oder geringeren Gehalten an Elektrolyten erzielt werden kann.

Sowohl in technischen als auch insbesondere in kosmetischen Anwendungen kommen die Betain-Zubereitungen auf Basis polymerer Fettsäuren nicht allein, sondern vorzugsweise in Gemischen mit anderen Wirkstoffen zum Einsatz. Als solche Wirkstoffe sind in erster Linie zu nennen:
(1) anionische Tenside, wie z.B. Fettalkoholsulfate, Fettalkoholethersulfate, Alkylsulfonate, Alkylbenzolsulfonate, Sulfobernsteinsäurealkylester, ferner
(2) nichtionische Tenside, wie z.B. Fettsäureglycerin- oder Sorbitanester, die gegebenenfalls zusätzlich alkoxyliert sein können, Fettalkohol- und Fettsäureethoxylate, Fettsäurealkanolamide, Polyalkylenglykole, Fettsäurezuckerester, Fettsäureglucosidester, Alkylglucoside, sowie
(3) amphotere Tenside, wie z.B. Betaine und Amphoglycinate und
(4) kationische Tenside.

Andere Wirk- und Zusatzstoffe in solchen Formulierungen können sein:
Kaolin, Bentonit, Fettsäuren, höhere Fettalkohole, Vaseline oder Paraffinöl, Verdickungsmittel, wie beispielsweise ethoxylierte Fettsäurederivate, Stärke, Polyacrylsäure und deren Derivate, Cellulosederivate, Alginate, ferner Trübungsmittel, wie Glykolesterderivate, Alkohole wie Ethanol, Propanol, Propylenglykol oder Glycerin, Lösungsvermittler, Stabilisatoren, Puffersubstanzen, Parfümöle, Farbstoffe sowie konditionierende und pflegende Bestandteile, wie z.B. kationische oder amphotere Polymere, Proteinhydrolysate, Lanolinderivate, Cholesterin, Pantothensäure, Polydimethylsiloxane oder deren Derivate.

Die erfindungsgemäßen Betain-Gemische eignen sich aufgrund ihres geringen Reizpotentials weiterhin zum Einsatz in Reinigungsmitteln, die während ihrer Anwendung mit der Haut in Berührung kommen, wie Handgeschirrspülmittel und andere Reinigungs-, Pflege- und Poliermittel.

In den folgenden Beispielen werden die Herstellung der Betain-Gemische und deren anwendungstechnische Eigenschaften noch näher gezeigt.

### I. Herstellung der als Ausgangsprodukte benötigten Amidamine (nicht erfindungsgemäß)

56 g Dimersäure (im Handel unter der Bezeichnung PRIPOL 1009 erhältlich) mit einer Säurezahl von 204 werden mit 26,4 g Dimethylaminopropylamin unter einem stetigen, langsamen Stickstoffstrom für etwa 4 Stunden erhitzt, wobei die Temperatur langsam von 140°C auf 200°C erhöht wird. Das entstandene Wasser wird ebenso wie das überschüssige Amin mit dem Stickstoffstrom ausgetragen. Die Reaktion ist abgeschlossen, wenn die Säurezahl des Reaktionsproduktes < 4 beträgt.

### II. Herstellung erfindungsgemäßer Betain-Gemische

### Beispiel II 1

34 g Dimersäureamidamin aus Dimersäure und Dimethylaminopropylamin und 28,8 g Cocosfettsäureamidamin aus Cocosfettsäure und Dimethylaminopropylamin werden auf 60°C erwärmt und mit einer Lösung von 19,2 g Natriumchloracetat in 343 g Wasser versetzt. Das Reaktionsgemisch wird 5 Stunden bei 95°C und einem pH-Wert > 8,5 gehalten. Durch Dünnschichtchromatographie wird ein Umsatz von > 95 % festgestellt. Das Produkt enthält 19,3 % Festkörper und 2,3 % Natriumchlorid. Die waschaktive Substanz (WAS) setzt sich zu gleichen Gewichtsteilen aus Dimersäurebetain und Cocosfettsäurebetain zusammen (jeweils 8,5 %). Das Produkt ist eine klare, schwach gelbliche, fließfähige, viskose, wäßrige Lösung mit schwach ausgeprägten viskoelastischen Eigenschaften.

In analoger Weise werden die Betain-Gemische II 2 bis II 5 hergestellt, die sich u.a. in ihrer Zusammensetzung unterscheiden. In der folgenden Tabelle 1 sind Zusammensetzung und Viskosität aufgeführt.

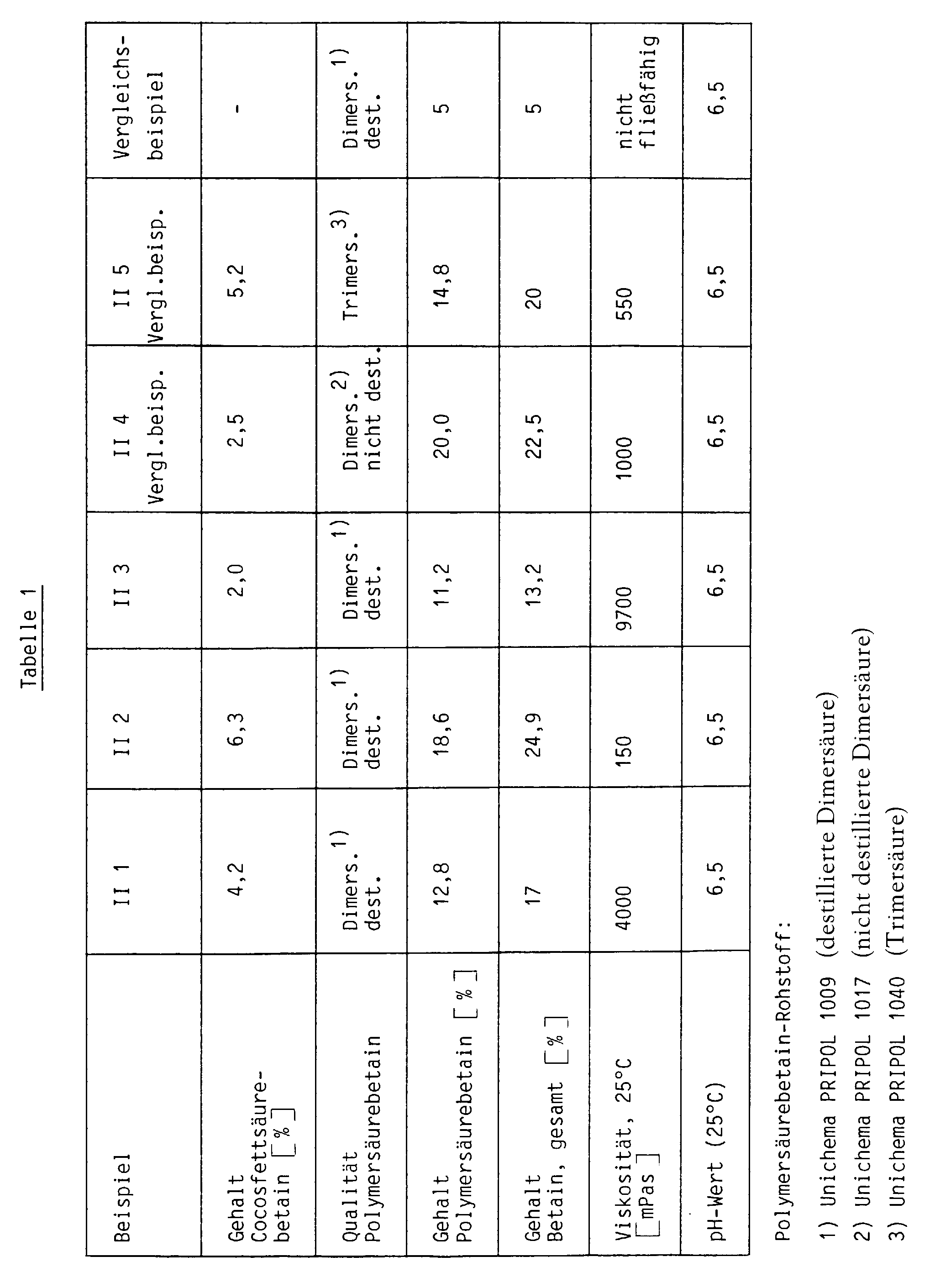

### III. Eigenschaften der erfindungsgemäßen Betain-Gemische

### Schaumverhalten

### Beispiel III 1

Zur Bestimmung des Schaumvermögens wird ein modifiziertes Ross-Miles-Verfahren gemäß DIN 53 902 angewandt. Bei dem Verfahren werden Auslaufzeit und Schaumhöhe 30 Sekunden, 3 und 5 Minuten nach dem freien Ausfließen von 500 ml einer Lösung der Probe in Wasser aus definierter Höhe auf die Flüssigkeitsoberfläche derselben Lösung gemessen.

Untersucht wird das Produkt gemäß Beispiel II 1. Außerdem wird das Schaumverhalten einer Mischung mit Natriumlaurylethersulfat (im Handel unter der Bezeichnung Texapon N 25 erhältlich) überprüft. Zum Vergleich wird das Schaumvermögen der reinen Tenside Cocosfettsäureamidopropylbetain und Natriumlaurylethersulfat angegeben.

Die Temperatur der zu untersuchenden Lösungen beträgt 45°C, die Tensidkonzentration 0,015 % WAS waschaktive Substanz. Die Ergebnisse sind in folgender Tabelle 2 zusammengefaßt.

**Tabelle 2**

| Produkt | Wasser 0° d.H. | | Wasser 16,8° d.H. | |
|---|---|---|---|---|
| | Zeit Min. | Schaumhöhe mm | Zeit Min. | Schaumhöhe mm |
| Produkt von Beispiel II 1 erfindungsgemäß | 0,5 | kein Schaum | 0,5 | kein Schaum |
| Cocosfettsäure | 0,5 | 100 | 0,5 | 100 |
| betain | 3 | 90 | 3 | 90 |
| nicht erfindungsgemäß | 5 | 85 | 5 | 85 |
| Prod. v. Beisp. II 1 | 0,5 | 100 | | |
| 0,003 % WAS + | 3 | 90 | | |
| Natriumlaurylethersulfat 0,012 % WAS | 5 | 88 | | |
| Natriumlaurylether | 0,5 | 140 | | |
| sulfat | 3 | 130 | | |
| nicht erfindungsgemäß | 5 | 127 | | |

Die Ergebnisse zeigen das geringe Schaumvermögen des Betain-Gemisches. Die Schaumbildung des anionischen Tensids Natriumlaurylethersulfat wird deutlich erniedrigt.

### Beispiel III 2

### Verbesserung der Haftlänge von Wolle

Die Haftlänge gibt Auskunft über das Faser/Faser-Reibungsverhalten.

| | |
|---|---|
| Substrat | Wolle, Kammzug |
| Feinheit | 21,7 µm |
| Länge | 67 mm |
| Prüfsubstanz | Betain-Gemisch gemäß Beispiel II 2. |

Die Bestimmung der Haftlänge erfolgt in Anlehnung an DIN 53 834: "Einfacher Zugversuch an Garnen und Zwirnen". Auf einen Kammzug werden 0,3 Gew.-% (bezogen auf Aktivsubstanz) des Betain-Gemisches aus wäßriger Verdünnung durch Sprühen appliziert. Nach Trocknen und Konditionieren bei 65% r.h., 20°C und 36 Stunden werden die Kammzugbänder zweimal auf einer Nadelstrecke zu Streckenbändern verstreckt. Die Streckenbänder weisen ein Gewicht von 9,17 g/m auf. Von den Streckenbändern werden Prüflinge von einer Länge von 200 mm (entspricht dreifacher Stapellänge) abgeschnitten. Diese Proben werden in einer Zwick-Universalprüfmaschine (Deutschland) einem Zugversuch mit einer Prüfgeschwindigkeit von 500 mm/min unterworfen. Der höchste Punkt der Kraft/ Dehnungskurve entspricht der maximalen Haftkraft und stellt die statische Faser/Faser-Reibung dar. Über das Gewicht des Streckenbandes wird aus der in diesem Versuch gemessenen Kraft die Länge (Haftlänge) berechnet, bei der das Streckenband durch sein Eigengewicht reißen würde (meter-Band-Haftung, mBH).

Als Ergebnisse (in mBH) werden erhalten:

| | |
|---|---|
| Wolle unbehandelt | 28,1 m, |
| Wolle behandelt mit Betain | 38,0 m. |

Durch die Anwendung des Betain-Gemisches wird die Haftlänge der Wolle deutlich verbessert.

### Beispiel III 3

### Shampooformulierung

Aus den folgenden Materialien wird ein Haarshampoo hergestellt:

| | |
|---|---|
| Natriumlaurylethersulfat ¹⁾ | 12,0 Gew.-% Aktivsubstanz |
| Cocosfettsäureamidopropylbetain ²⁾ | 3,0 Gew.-% Aktivsubstanz |
| Betain-Gemisch gemäß Beispiel II 2 | 4,0 Gew.-% Aktivsubstanz |
| NaCl | 0,5 Gew. -% |
| Wasser | 80,5 Gew.-% |

| | |
|---|---|
| ¹⁾ aus Texapon N 25 | |
| ²⁾ aus TEGO Betain L7 | |

Die Bestandteile werden in der angegebenen Reihenfolge zusammengegeben.

Die Formulierung ist klar, leicht gelblich und weist eine Viskosität von 7500 mPas und einen pH-Wert von 6,5 auf. Mit dieser Shampooformulierung gewaschene Haare erhalten einen angenehmen Griff und eine gute Frisierbarkeit, die deutlich gegenüber solchen Haaren verbessert ist, die mit einer vergleichbaren Shampooformulierung ohne Dimersäurebetain gewaschen wurden.

## Patentansprüche

1. Wäßrige, fließfähige Zubereitung aus Betainen auf der Basis von Fettsäureamiden und Polymersäureamiden, gekennzeichnet durch einen Gehalt von
5 bis 30 Gew.-% eines Gemisches von Betainen, welches aus 15 bis 60 Gew.-% Betainen der allgemeinen Formel 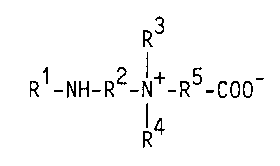 und
85 bis 40 Gew.-% Betainen der allgemeinen Formel 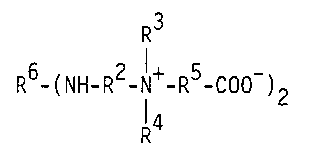 besteht und wobei die Gew.-% sich zu 100 Gew.-% ergänzen, wobei
R¹ der Acylrest einer Fettsäure mit 8 bis 18 Kohlenstoffatomen oder eines Gemisches dieser Fettsäuren ist,
R² ein zweiwertiger aliphatischer Kohlenwasserstoffrest mit 2 bis 5 Kohlenstoffatomen ist,
R³, R⁴ unabhängig voneinander aliphatische Kohlenwasserstoffreste mit 1 bis 4 Kohlenstoffatome sind,
R⁵ der Rest -CH₂- oder -(CH₂)₂- ist,
R⁶ ein von destillierter Dimersäure abgeleiteter Acylrest ist,
95 bis 70 Gew.-% Wasser, Alkalisalze und gegebenenfalls übliche Zusatzstoffe wie Hilfslösungsmittel, Konservierungsmittel oder dergl.

2. Zubereitung nach Anspruch 1, dadurch gekennzeichnet, daß der Rest R² ein -(CH₂)₂- oder -(CH₂)₃-Rest ist.

3. Zubereitung nach Anspruch 1, dadurch gekennzeichnet, daß die Reste R³ und R⁴ Methylreste sind.

4. Zubereitung nach Anspruch 1, dadurch gekennzeichnet, daß der Rest R⁵ ein -CH₂-Rest ist.

5. Zubereitung nach Anspruch 1, dadurch gekennzeichnet, daß der Rest R¹ ein von dem Cocosfettsäuregemisch abgeleiteter Acylrest ist.

6. Verwendung einer Zubereitung nach Anspruch 1, gegebenenfalls in Abmischung mit anderen Tensiden, als Wasch- und Reinigungsmittel zur Körperpflege, als Hilfsmittel in der Textilindustrie, und als Reinigungsmittel für technische Zwecke.

## Claims

1. Aqueous, free-flowing preparation comprising betaines based on fatty acid amides and polymeric acid amides, characterized by a content of
5 to 30 % by weight of a mixture of betaines which consists of
15 to 60 % by weight of betaines of the general formula 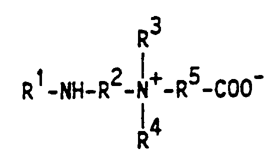 and
85 to 40 % by weight of betaines of the general formula 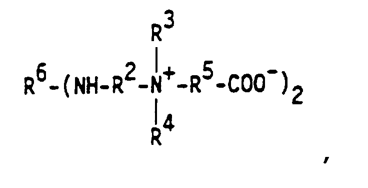 the % by weight adding up to 100 % by weight, where
R¹ is the acyl radical of a fatty acid having from 8 to 18 carbon atoms or a mixture of such fatty acids,
R² is a divalent aliphatic hydrocarbon radical having from 2 to 5 carbon atoms,
R³, R⁴ independently of one another are aliphatic hydrocarbon radicals having from 1 to 4 carbon atoms,
R⁵ is the -CH₂- radical or -(CH₂)₂-,
R⁶ is an acyl radical derived from distilled dimer acid, and
95 to 70 % by weight of water, alkali metal salts and, if required, customary additives such as auxiliary solvents, preservatives or the like.

2. Preparation according to Claim 1, characterized in that the R² radical is a -(CH₂)₂- or -(CH₂)₃- radical.

3. Preparation according to Claim 1, characterized in that the R³ and R⁴ radicals are methyl radicals.

4. Preparation according to Claim 1, characterized in that the R⁵ radical is a -CH₂- radical.

5. Preparation according to Claim 1, characterized in that the R¹ radical is an acyl radical derived from the coconut fatty acid mixture.

6. Use of a preparation according to Claim 1, if required mixed with other surfactants, as detergents and cleaners for body care, as auxiliaries in the textile industry and as cleaners for industrial purposes.

## Revendications

1. Préparation aqueuse, fluide, de bétaïnes à base d'amides gras et de polyamides, caractérisée en ce qu'elle contient :
5 à 30% en poids d'un mélange de bétaïnes, qui consiste en
15 à 60% en poids de bétaïnes de formule générale : 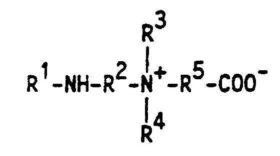 et
85 à 40% en poids de bétaïnes de formule générale : 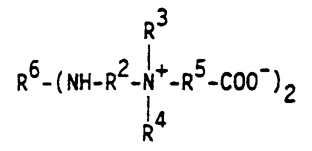 les % en poids se complétant à 100% en poids, dans lesquelles :
R¹ est le radical acyle d'un acide gras ayant 8 à 18 atomes de carbone ou d'un mélange de ces acides gras;
R² est un radical hydrocarboné aliphatique divalent ayant 2 à 5 atomes de carbone;
R³, R⁴ sont indépendamment l'un de l'autre des radicaux hydrocarbonés aliphatiques ayant 1 à 4 atomes de carbone;
R⁵ est le radical -CH₂- ou -(CH₂)₂-, et
R⁶ est un radical acyle dérivé d'un acide dimère distillé;
95 à 70% en poids d'eau, de sels alcalins et éventuellement d'additifs courants, comme un agent d'aide à la solubilisation, un agent conservateur ou des produits semblables.

2. Préparation suivant la revendication 1, caractérisée en ce que le radical R² est un radical -(CH₂)₂ ou -(CH₂)₃-.

3. Préparation suivant la revendication 1, caractérisée en ce que les radicaux R³ et R⁴ sont des radicaux méthyle.

4. Préparation suivant la revendication 1, caractérisée en ce que le radical R⁵ est un radical -CH₂-.

5. Préparation suivant la revendication 1, caractérisée en ce que le radical R¹ est un radical acyle dérivé du mélange d'acides gras de coco.

6. Utilisation d'une préparation suivant la revendication 1, éventuellement mélangée avec d'autres surfactants, comme produit de lavage ou comme produit de nettoyage pour les soins corporels, comme auxiliaire dans l'industrie textile, et comme produit de nettoyage pour des fonctions techniques.
